# EUROPEAN PATENT APPLICATION

(11) **EP 4 759 376 A1**
(43) Date of publication of application: **17.06.2026**
(21) Application number: 25218230.8
(22) Date of filing: 25.11.2025
(51) Int. Cl.: A61Q 5/12, A61K 8/06, A61K 8/92, A61K 8/9789, A61K 8/9794

(54) **HAIR COSMETIC EMULSION COMPRISING A BOTANICAL MIXTURE COMPRISING 9 BOTANICAL COMPONENTS**

(30) Priority: 12.12.2024 EP 24219429
(71) Applicant: Wella Germany GmbH, 64295 Darmstadt (DE)
(72) Inventor: Akhila, Nair, 64295 Darmstadt (DE)
(74) Representative: Ziebig Hengelhaupt Intellectual Property Attorneys Patentanwaltskanzlei PartGmbB

(57) **Abstract**

The presently claimed invention relates to a hair cosmetic emulsion comprising an aqueous phase (AP) forming the continuous phase of the hair cosmetic emulsion, and an oil phase (OP) forming the disperse phase of the hair cosmetic emulsion, wherein the hair cosmetic emulsion comprises a botanical mixture (BM) comprising the following 9 botanical components: bamboo extract, cactus extract, jojoba oil, safflower oil, olive oil, babassu oil, sesame seed oil, rice bran oil, and evening primrose oil. The presently claimed invention, moreover, is drawn to a method for treating human hair with the hair cosmetic emulsion, and the use of the hair cosmetic emulsion for treating human hair, especially for nourishing and/or strengthening human hair, or protecting human hair against pollution.

## Description

### Field of the invention

The presently claimed invention relates to a hair cosmetic emulsion comprising an aqueous phase (AP) forming the continuous phase of the hair cosmetic emulsion, and an oil phase (OP) forming the disperse phase of the hair cosmetic emulsion, wherein the hair cosmetic emulsion comprises a botanical mixture (BM) comprising the following 9 botanical components: bamboo extract, cactus extract, jojoba oil, safflower oil, olive oil, babassu oil, sesame seed oil, rice bran oil, and evening primrose oil. The presently claimed invention, moreover, is drawn to a method for treating human hair with the hair cosmetic emulsion, and the use of the hair cosmetic emulsion for treating human hair, especially for nourishing and/or strengthening human hair, or protecting human hair against pollution.

### Background of the invention

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with the combing and result in a condition commonly referred to as "fly-away hair" or contribute to an undesirable phenomenon of "split ends".

Further, chemical treatments, such as perming, bleaching, or coloring hair, can also damage hair and leave it dry, rough, lusterless, and damaged. The hair, moreover, can be damaged by ultraviolet radiation or heat.

A variety of approaches have been developed to condition the hair. A common method of providing conditioning benefits to the hair is through the use of conditioning agents such as cationic surfactants and high melting point fatty compounds, silicone compounds, and mixtures thereof.

Especially silicone compounds have been excessively used over the past decades to provide conditioners with enhanced benefits such as hair shine, softness, dry hair smoothness, hair strand alignment (e.g. minimize frizziness), and ease of combing.

However, consumers are becoming more aware of the safety of the ingredients used in their cosmetic products, in particular, the use of synthetic compounds whose toxicology and environmental impact is questionable.

Accordingly, there is a need for keratin fiber treatments which are free of components whose toxicology and environmental impact is questionable.

Moreover, there is a need for hair cosmetic products that can protect keratin fibers, especially human hair, especially against heat up to 230°C, against ultraviolet radiation, and against pollution buildup on the hair.

### Summary of the invention

It is an object of the presently claimed invention to provide a composition that is capable to condition and protect human hair. The composition for conditioning and protecting should preferably be capable to protect keratin fibers, especially human hair, against heat up to 230°C, against ultraviolet radiation, and/or against pollution buildup on the hair.

The object has been solved by a hair cosmetic emulsion comprising a botanical mixture (BM) comprising 9 botanical components.

Accordingly, in one aspect, the presently claimed invention is directed to a hair cosmetic emulsion comprising
an aqueous phase (AP) forming the continuous phase of the hair cosmetic emulsion, and
an oil phase (OP) forming the disperse phase of the hair cosmetic emulsion,
wherein the hair cosmetic emulsion comprises a botanical mixture (BM) comprising the following 9 botanical components
   - b1): bamboo extract,
   - b2): cactus extract,
   - b3): jojoba oil,
   - b4): safflower oil,
   - b5): olive oil,
   - b6): babassu oil,
   - b7): sesame seed oil,
   - b8): rice bran oil, and
   - b9): evening primrose oil.

Surprisingly, it was found that the hair cosmetic emulsion comprising the botanical mixture (BM) is capable of protecting and conditioning human hair. Moreover, it was surprisingly found that the hair cosmetic emulsion comprising the botanical mixture (BM) provides one, two or more, or all of the following advantages which are, heat protection up to 230°C, protection against ultraviolet radiation, protection from hair breakage, protection from hair split ends, protection from pollution buildup on the hair, and protection from hair frizz.

The hair cosmetic emulsion comprising the botanical mixture (BM), moreover, is capable to nourish and moisturize human hair, and to act as moisturize repelling shield.

The present invention is effective for providing hair protection and conditioning benefits to hair that is damaged by natural, environmental factors such as shampooing, as well as chemical hair treatments such as bleaching, coloring, or perming.

### Detailed description of the invention

### Botanical mixture (BM)

The botanical mixture (BM), comprised in the hair cosmetic emulsion, comprises the following 9 botanical components
- b1): bamboo extract,
- b2): cactus extract,
- b3): jojoba oil,
- b4): safflower oil,
- b5): olive oil,
- b6): babassu oil,
- b7): sesame seed oil,
- b8): rice bran oil, and
- b9): evening primrose oil.

In one embodiment the botanical mixture (BM), comprised in the hair cosmetic emulsion, consists of the 9 botanical components b1), b2), b3), b4), b5), b6), b7), b8), and b9).

The 9 botanical components b1), b2), b3), b4), b5), b6), b7), b8), and b9) are described in more detail hereinafter.

In a preferred embodiment, the botanical mixture (BM) offers versatile benefits tailored to different hair types, making it suitable for a wide range of users. For dry and damaged hair, the oils and extracts provide deep hydration, repair split ends, and restore lost elasticity, leaving hair softer and more manageable. Oily hair benefits from lightweight, non-greasy hydration and scalp-balancing properties that help regulate sebum production without clogging pores. For curly or textured hair, the formulation enhances moisture retention, reduces frizz, and defines curls, providing smoothness and control. Fine hair experiences volumizing effects as the lightweight nature of the ingredients avoids weighing down strands while still providing necessary nourishment. Additionally, for chemically treated or color-treated hair, the antioxidants and reparative compounds protect against further damage, prevent color fading, and reinforce hair strength. This multi-functional approach ensures that the mixture caters to diverse hair needs, enhancing overall health and appearance.

### b1) bamboo extract:

Bamboo extract (component b1)) is an extract derived from any part of a bamboo plant in the Bambusa genus. Preferably, component b1) is extracted from the leaves, stems, and/or shoots of a Bambusa species, such as preferably Bambusa vulgaris, ensuring a rich concentration of bioactive compounds. Other suitable species of Bambusa in addition to Bambusa vulgaris are Bambusa oldhamii, Bambusa balcooa, Bambusa bambos, Bambusa tulda, Bambusa textilis, Bambusa multiplex, Bambusa arundinacea, Bambusa nutans, Bambusa blumeana, Bambusa tuldoides, Bambusa pervariabilis, Bambusa emeiensis, Bambusa lako, Bambusa dissimulator, Bambusa ventricosa and Bambusa beecheyana.

Examples for suitable extracting agents are water, alcohols and water-alcohol mixtures. Preferred alcohols are C₁₋₆-alcohols or mixtures of two or more thereof, more preferably linear C₁₋₆-alcohols, yet more preferably C₁₋₄-alcohols, even more preferably linear C₁₋₄-alcohols, still more preferably methanol, ethanol, n-propanol or isopropanol and most preferably ethanol. Preferred water-alcohol mixtures are mixtures containing 30 to 70% by volume of a C₁₋₆-alcohol, more preferably a C₁₋₄-alcohol, even more preferably a C₁₋₃-alcohol and most preferably ethanol.

It is particularly preferred that component b1) is obtained through an optimized aqueous or hydroalcoholic extraction process, preserving its active constituents such as flavonoids, amino acids, phenolic acids, and polyphenols. These compounds work synergistically to deliver multiple benefits: Flavonoids (e.g., orientin, isoorientin) provide potent antioxidant properties that protect hair from oxidative stress caused by environmental factors like UV exposure and pollution. Amino Acids (e.g., serine, glycine, proline) help repair and fortify the hair structure while enhancing scalp hydration and nourishment. Phenolic Acids and Polyphenols exhibit anti-inflammatory and soothing effects, making the extract suitable for sensitive or irritated scalps, while supporting overall scalp health and reducing dandruff.

### b2) cactus extract:

Cactus extract (component b2)) is an extract derived from any part of a cactus plant, such as from pads, stems, flowers and/or fruits, within the plant family Cactaceae, renowned for its hydrating and protective properties. Suitable genera of Cactaceae are Opuntia, Cereus, Carnegiea, Pachycereus, Stenocereus, Neobuxbaumia, Pilosocereus, Cephalocereus, Stephanocereus, Armatocereus, Lemaireocereus, Ferocactus, Echinocactus, Thelocactus, *Parodia,* Echinopsis, Gymnocalycium, Mammillaria, Rebutia, Astrophytum, Lophophora, Cylindropuntia, Tephrocactus, Austrocylindropuntia, Maihuenia, Epiphyllum, Schlumbergera, Hatiora, Ariocarpus, Turbinicarpus and Blossfeldia.

Examples for suitable extracting agents are water, alcohols and water-alcohol mixtures. Preferred alcohols are C₁₋₆-alcohols or mixtures of two or more thereof, more preferably linear C₁₋₆-alcohols, yet more preferably C₁₋₄-alcohols, even more preferably linear C₁₋₄-alcohols, still more preferably methanol, ethanol, n-propanol or isopropanol and most preferably ethanol. Preferred water-alcohol mixtures are mixtures containing 30 to 50% by volume of a C₁₋₆-alcohol, more preferably a C₁₋₄-alcohol, even more preferably a C₁₋₃-alcohol and most preferably ethanol.

Preferably, component b2) is extracted from the pads, stems, flowers and/or fruits of Opuntia ficus-indica (prickly pear cactus), which is particularly rich in bioactive compounds, or *Cereus Grandiflorius.* Component b2) is produced through advanced water, ethanol, or mixed solvent extraction processes, designed to maximize the preservation of its beneficial constituents. These include: Polysaccharides (e.g., mucilage) provide superior hydration by forming a moisture-retentive barrier on both hair and scalp, which is especially effective in dry or arid conditions. Vitamins (e.g., vitamin E, vitamin C, and B vitamins) deliver powerful antioxidant protection against free radicals, helping to prevent environmental damage and oxidative stress on the hair and scalp. Minerals (e.g., calcium, magnesium, potassium) support overall scalp health and contribute to strengthening hair roots, reducing hair fall. Amino Acids (e.g., proline, taurine) enhance scalp hydration, repair damage, and promote elasticity in hair strands, minimizing brittleness.

### b3) jojoba oil:

Jojoba oil (component b3)) is preferably obtained from the seeds of Simmondsia chinensis, commonly in the form of a liquid wax ester.

Component b3) preferably comprises primarily long-chain monounsaturated fatty acids (e.g., eicosenoic acid) and long-chain alcohols (e.g., docosanol, tetracosanol). Additionally, it is preferably rich in tocopherols (vitamin E), which provide antioxidant properties, and contains phytosterols that contribute to soothing and protecting the skin.

The production of component b3) (jojoba oil) preferably involves cold pressing the seeds of Simmondsia chinensis, followed by filtration to ensure purity.

### b4) safflower oil:

Safflower oil (component b4)) is preferably obtained from the seeds of Carthamus tinctorius.

Component b4) predominantly comprises linoleic acid (up to 75%), an omega-6 fatty acid, along with oleic acid and smaller proportions of palmitic and stearic acids. It is preferably enriched with vitamin E (tocopherols) and phytosterols (such as β-sitosterol, campesterol, and stigmasterol), which provide anti-inflammatory and moisturizing benefits.

The production of component b4) (safflower oil) preferably involves cold pressing or solvent extraction methods.

Jojoba oil and safflower oil preferably work synergistically to ensure optimal moisture retention in hair and scalp care. Jojoba oil, a liquid wax ester derived from Simmondsia chinensis, closely mimics the natural sebum of the scalp, creating a lightweight, non-greasy barrier that locks in moisture and prevents dehydration. Its rich content of monounsaturated fatty acids and tocopherols enhances scalp health while smoothing the hair cuticle. Complementing this, safflower oil, derived from Carthamus tinctorius, is high in linoleic acid, an omega-6 fatty acid, which penetrates the hair shaft to restore lipid balance and reinforce the hair's structural integrity. Together, these oils form a dual-action system where jojoba oil seals and protects the hair's outer layer, while safflower oil deeply nourishes and hydrates from within, resulting in soft, resilient, and well-moisturized hair. Their combined action not only prevents moisture loss but also enhances overall scalp health, making this pairing ideal for maintaining hydration in various hair types.

### b5) olive oil:

Olive oil (component b5)) is preferably obtained from the fruits of Olea europaea.

Component b5) primarily comprises monounsaturated fatty acids, with oleic acid (55-83%) as the major component, along with smaller amounts of linoleic acid, palmitic acid, and stearic acid. It preferably contains phenolic compounds (e.g., hydroxytyrosol and oleuropein), squalene, tocopherols (vitamin E), and phytosterols, which provide antioxidant protection, emollient properties, and conditioning benefits, making it well-suited for applications in hair and skin care.

The production of component b5) (olive oil) preferably involves cold pressing or solvent extraction techniques.

### B6) babassu oil:

Babassu oil (component b6)) is preferably obtained from the seeds of Attalea speciosa (babassu palm).

Component b6) primarily consists of medium-chain fatty acids, with lauric acid (up to 50%) as the major component, along with myristic acid, palmitic acid, and stearic acid. It also preferably contains small quantities of phytosterols (e.g., β-sitosterol, stigmasterol) and tocopherols (vitamin E), making it a versatile emollient that effectively provides moisture without leaving a greasy residue.

The production of component b6) (babassu oil) preferably involves mechanical pressing or solvent extraction methods.

### b7) sesame seed Oil:

Sesame seed oil (component b7)) is preferably obtained from the seeds of Sesamum indicum.

Component b7) primarily contains oleic acid (35-50%) and linoleic acid (35-50%), along with smaller amounts of palmitic acid, stearic acid, and arachidic acid. It is distinguished by its antioxidant components, including lignans (such as sesamin, sesamolin, and sesaminol), tocopherols (vitamin E), and phytosterols (e.g., β-sitosterol, campesterol), which enhance its stability and nourishing properties.

The production of component b7) (sesame seed oil) preferably involves cold pressing or solvent extraction processes.

### b8) rice bran oil:

Rice bran oil (component b8)) is preferably obtained from the bran layer of Oryza sativa (rice).

Component b8) features a balanced fatty acid profile, predominantly oleic acid (up to 50%), linoleic acid (up to 35%), and palmitic acid. It is notably rich in gamma oryzanol, a distinctive antioxidant comprising a mixture of ferulic acid esters of sterols and triterpene alcohols. Additionally, component b8) preferably includes tocopherols and tocotrienols (vitamin E), squalene, and phospholipids, which enhance its antioxidant properties, amplify conditioning effects, and improve smoothing benefits.

The production of component b8) (rice bran oil) preferably involves cold pressing or solvent extraction techniques.

### b9) evening primrose oil:

Evening primrose oil (component b9)) is preferably obtained from the seeds of Oenothera biennis.

Component b9) is characterized by a high concentration of gamma-linolenic acid (GLA), an omega-6 fatty acid, along with significant amounts of linoleic acid, oleic acid, and palmitic acid. It preferably also contains phytosterols (e.g., β-sitosterol) and tocopherols (vitamin E), which contribute to its anti-inflammatory and moisturizing properties.

The production of component b9) (evening primrose oil) is preferably achieved through cold pressing.

In a preferred embodiment the botanical mixture (BM) comprises
1.0 to 10.0 % by weight of component b1),
1.0 to 10.0 % by weight of component b2),
10.0 to 30 % by weight of component b3),
10.0 to 30 % by weight of component b4),
5.0 to 15 % by weight of component b5),
5.0 to 15 % by weight of component b6),
5.0 to 15 % by weight of component b7),
5.0 to 15 % by weight of component b8),
5.0 to 15 % by weight of component b9),
wherein the % by weight values are based on the total weight of the components b1), b2), b3), b4), b5), b6), b7), b8), and b9) comprised in the botanical mixture (BM), wherein the sum of the % by weight values of the components b1), b2), b3), b4), b5), b6), b7), b8), and b9) is 100 % by weight.

Preferably the % by weight values are based on the total weight of the botanical mixture (BM), wherein the sum of the % by weight values of the components b1), b2), b3), b4), b5), b6), b7), b8), and b9) is less than or equal to 100 % by weight. If the sum of components b1) to b9) is 100 % by weight of the botanical mixture (BM), the botanical mixture (BM) consists of the components b1), b2), b3), b4), b5), b6), b7), b8), and b9), whereas in the case that the sum of components b1) to b9) is less than 100 % by weight of the botanical mixture (BM), the botanical mixture (BM) comprises the components b1), b2), b3), b4), b5), b6), b7), b8), and b9) and one or more further components.

### Hair cosmetic emulsion

The hair cosmetic emulsion according to the invention comprises
an aqueous phase (AP) forming the continuous phase of the hair cosmetic emulsion, and
an oil phase (OP) forming the disperse phase of the hair cosmetic emulsion.

In a preferred embodiment of the present invention the hair cosmetic emulsion comprises 0.5 to 5 % by weight, more preferred 0.5 to 2.0 % by weight, and particularly preferred 0.5 to 1.6 % by weight of the botanical mixture (BM) based on the total weight of the hair cosmetic emulsion.

In a preferred embodiment of the present invention the botanical components b1) and b2) are comprised in the aqueous phase (AP) of the hair cosmetic emulsion and the botanical components b3), b4), b5), b6), b7), b8), and b9) are comprised in the oil phase (OP) of the hair cosmetic emulsion.

### aqueous phase (AP)

In preferred embodiment of the present invention the aqueous phase (AP) of the hair cosmetic emulsion comprises water and at least one cosmetically acceptable organic medium.

The term "at least one cosmetically acceptable organic medium" means exactly one cosmetically acceptable organic medium and, also a mixture of two or more different cosmetically acceptable organic mediums.

In a preferred embodiment of the present invention the aqueous phase (AP) of the hair cosmetic emulsion comprises water in an amount in the range of 70 to 90 % by weight based on the total weight of the aqueous phase (AP) comprised in the hair cosmetic emulsion.

In another embodiment the aqueous phase (AP) of the hair cosmetic emulsion comprises the cosmetically acceptable organic medium in an amount in the range of 10 to 20 % by weight based on the total weight of the aqueous phase (AP) comprised in the hair cosmetic emulsion.

The cosmetically acceptable organic medium is preferably a water miscible organic solvent, preferably at least one alcohol.

In another embodiment the cosmetically acceptable organic medium is at least one selected from the group consisting of a monoalcohols, such as ethanol and isopropanol, and polyalcohols, such as propylene glycol, pentylen glycol, hexylene glycol, and glycerin.

In a preferred embodiment the aqueous phase (AP) of the hair cosmetic emulsion comprises water in an amount in the range of 70 to 90 % by weight and a mixture of pentylen glycol, propandiol, and glycerin in an amount in the range of 10 to 20 % by weight, based on the total weight of the aqueous phase (AP) comprised in the hair cosmetic emulsion.

The viscosity of the aqueous phase (AP) can be controlled in particular by acting on the amount of anionic polymer (carbomer in particular) and the pH of the aqueous phase (AP). In general, the pH of the aqueous phase (AP) is in the range between 5.5 and 6.5.

In a preferred embodiment the hair cosmetic emulsion according to the invention the pH value of the aqueous phase (AP) is in the range of 5.5 to 6.5.

In one embodiment, the aqueous phase (AP) has a viscosity between 400 mPas and 100 000 mPas, preferably between 800 mPas and 30 000 mPas, measured at 25° C. The viscosity is measured using the method described hereinafter.

Unless otherwise stated in the remainder hereof, the temperature is considered to be ambient temperature (e.g. T=25 °C ± 2 °C) and pressure is atmospheric pressure.

The viscosity is measured at ambient temperature, for example T=25°, and at atmospheric pressure, by the method described below.

A Brookfield type viscometer, typically a Brookfield RVDV-E digital viscometer (spring twist torque of 7187.0 dyne-cm), i.e. a rotational speed viscometer equipped with a spindle, is used. A rotational speed is imposed on the spindle and the measurement of the torque exerted on the spindle makes it possible to determine the viscosity by knowing the geometry/shape parameters of the spindle used. For example, a spindle of size No. 04 (Brookfield reference: RV4) is used. The shear rate corresponding to the measurement of the viscosity is defined by the spindle used and the speed of rotation thereof.

The viscosity measurement is carried out for 1 minute at room temperature (T=25 °C ±2 °C). About 150 g of solution are placed in a beaker of 250 ml volume, having a diameter of about 7 cm so that the height of the volume occupied by the 150 g of solution is sufficient to reach the level marked on the spindle. Then, the viscometer is started at a speed of 10 rpm until the value displayed on the screen becomes stable. This measurement gives the viscosity of the tested fluid, as mentioned in the context of the present invention.

As previously indicated, the aqueous phase (AP) of the hair cosmetic emulsion of the invention the is preferably in gel form. Advantageously, the aqueous phase (AP) is not solid at ambient temperature (25 °C) and atmospheric pressure i.e. it is able to flow under its own weight. In one embodiment, the aqueous phase (AP) has viscosity of between 400 mPas and 100 000 mPas, preferably between 800 mPas and 30 000 mPas, such as measured at 25° C. This viscosity is measured using the above-described method.

The aqueous phase (AP)of the hair cosmetic emulsion of the invention may also comprise at least one base. It may comprise a single base or a mixture of several different bases. The presence of at least one base in the aqueous phase (AP) contributes in particular to enhancing the viscosity of the aqueous phase (AP). In one embodiment, the base contained in the aqueous phase (AP) is a mineral base. In one embodiment, the mineral base is selected from the group formed of alkali metal hydroxides and alkaline-earth metal hydroxides.

Preferably, the mineral base is a hydroxide of alkali metals and NaOH is particularly preferred.

In one embodiment, the base contained in the aqueous phase (AP) is an organic base. Among organic bases, mention can be made for example of ammonia, pyridine, triethanolamine, aminomethyl propanol, or triethylamine.

The hair cosmetic emulsion according to the invention can comprise from 0.01 to 10 weight %, preferably from 0.01 to 5 weight %, and more preferably from 0.02 to 1 weight % of base, preferably of mineral base and NaOH in particular, relative to the total weight of the hair cosmetic emulsion.

In a preferred embodiment the aqueous phase (AP) of the hair cosmetic emulsion comprises at least one anionic polymer.

The term "at least one anionic polymer" means exactly one anionic polymer and, also a mixture of two or more different anionic polymers.

The anionic polymer acts as a thickener to increase the viscosity of the aqueous phase (AP).

In the present invention, by "anionic polymer" it is meant a polymer comprising chemical functions of anionic type. By "chemical function of anionic type", it is meant a chemical function AH capable of losing a proton to give a function A⁻. Depending on the conditions of the medium in which it is contained, a polymer of anionic type therefore comprises chemical functions in AH form, or else in the form of its deprotonated base A⁻.

As examples of chemical functions of anionic type, mention can be made of the carboxylic acid functions -COOH, which may be present in the form of a carboxylate anion -COO⁻.

As examples of polymers of anionic type, mention can be made of any polymer formed by polymerization of monomers of which at least one part carries chemical functions of anionic type such as carboxylic acid functions. For example, such monomers are acrylic acid, maleic acid, or any ethylenically unsaturated monomer comprising at least one carboxylic acid function.

Preferably, the anionic polymer is hydrophilic i.e. soluble or dispersible in water.

Among the examples of polymers of anionic type suitable for implementing the invention, mention can be made of copolymers of acrylic acid or maleic acid with other monomers such as acrylamide, alkyl acrylates, C₅-C₈ alkyl acrylates, C₁₀-C₃₀ alkyl acrylates, C₁₂-C₂₂ alkyl methacrylates, methoxypolyethyleneglycol methacrylates, hydroxyester acrylates, crosspolymer acrylates, and mixtures thereof.

In a preferred embodiment of the present invention the anionic polymer is at least one carbomer as described below. The carbomer can also be a crosslinked acrylates/C₁₀₋₃₀ alkyl acrylate copolymer (INCI name: acrylates/C₁₀₋₃₀ alkyl acrylate crosspolymer).

In the invention and unless otherwise stated, by "carbomer" it is meant an optionally crosslinked homopolymer derived from polymerization of acrylic acid. It is therefore an optionally crosslinked poly(acrylic acid). Among the carbomers of the invention mention can be made of those marketed under the trade names Tego^{®} Carbomer 340FD by Evonik, Carbopol^{®} 981 by Lubrizol, Carbopol ETD 2050 by Lubrizol, or Carbopol Ultrez 10 by Lubrizol.

In one embodiment, by "carbomer" or "Carbopol^{®}" it is meant an acrylic acid polymer of high molecular weight crosslinked with allyl sucrose or allyl ethers of pentaerythritol (Handbook of Pharmaceutical Excipients, 5th Edition, pill). For example, it is Carbopol^{®}910, Carbopol^{®}934, Carbopol^{®}934P, Carbopol^{®}940, Carbopol^{®}941, Carbopol^{®}71 G, Carbopol^{®}980, Carbopol^{®}971P or Carbopol^{®}974P. In one embodiment, the viscosity of said carbomer is between 4 000 and 60 000 cP at 0.5% w/w.

Carbomers are also known under other names which are: polyacrylic acids, carboxyvinyl polymers or carboxy polyethylenes.

The aqueous phase (AP) according to the invention may comprise from 0.01 to 5 weight %, preferably 0.05 to 2 weight %, and more preferably 0.1 to 0.5 weight % of anionic polymer(s), of carbomers in particular, relative to the total the aqueous phase (AP).

The aqueous phase (AP) according to the invention can comprise a carbomer and an acrylates/C₁₀₋₃₀ alkyl acrylate Crosspolymer.

The aqueous phase (AP) of the invention can also comprise at least one crosslinked polymer or at least one crosslinked copolymer, said crosslinked polymer or crosslinked copolymer comprising at least one unit derived from the polymerization of one of the following monomers: acrylic or methacrylic acid, alkyl acrylate or methacrylate having 1 to 30 carbon atoms, or the salts thereof
In a preferred embodiment the aqueous phase (AP) of the hair cosmetic emulsion comprises at least one polyvinylpyrrolidone thickener in an amount in the range of 0.1 to 1.5 wt.-% based on the total weight of the aqueous phase (AP) comprised in the hair cosmetic emulsion. Preferably a polyvinylpyrrolidone of the K-90 typ.

### Oil phase (OP)

In a preferred embodiment of the present invention the oil phase (OP) comprised in the hair cosmetic emulsion is in the form of droplets.

The drops of the hair cosmetic emulsion of the present invention are preferably macroscopic droplets i.e. said droplets are visible to the naked eye as opposed to microscopic droplets that are not visible to the naked eye.

Therefore, in one embodiment, at least 60%, even at least 70%, preferably at least 80% and more preferred at least 90% of the drops have a mean diameter D larger than or equal to 100 µm, even larger than or equal to 200 µm, better still larger than or equal to 300 µm, in particular larger than or equal to 400 µm.

In a preferred embodiment the mean diameter D of the droplets in the range between 100 µm and 3000 µm, better still between 200 µm and 2000 µm, in particular between 300µm and 1700 µm, better still between 400 µm and 1600 µm, and most preferred in the range of preferably between 450 µm and 1550 µm and most particular preferred between 500 µm and 1500 µm.

Advantageously, in a hair cosmetic emulsion according to the invention, the droplets having a diameter in the range between 100 µm and 3000 µm, better still between 200 µm and 2000 µm, in particular between 300µm and 1700 µm, better still between 400 µm and 1650 µm, and most preferred in the range of preferably between 450 µm and 1550 µm and most particular preferred between 500 µm and 1500 µm represent a volume of 60% or more, even 70% or more, preferably 80% or more and better still of 90% or more of the total volume of the oil phase (OP).

Determination of the volume of droplets having a particular diameter, as mentioned above, relative to the total volume of the oil phase (OP) lies within the general knowledge of persons skilled in the art.

In addition, the droplets have advantageously apparent monodispersity (i.e. they are seen as spheres of identical diameter). Advantageously, the droplets are substantially spherical.

The hair cosmetic emulsion of the present invention can be prepared using different methods. Therefore, the hair cosmetic emulsions of the present invention preferably have the advantage of being able to be prepared with a simple "non-microfluidic" method, namely by mere emulsification.

As with conventional emulsions, for the preparation of the hair cosmetic emulsion according to the invention, the aqueous phase (AP) and the oil phase (OP) are preferably prepared separately. The hair cosmetic emulsion, in one embodiment, is prepared by addition of the oil phase (OP) to aqueous phase (AP) under agitation, whereby the hair cosmetic emulsion is obtained.

The viscosity of the aqueous phase (AP) and the shear force applied to the mixture are the two chief parameters which impact the size and monodispersity of the droplets comprised in the hair cosmetic emulsion. Skilled persons are able to adjust the non-microfluidic method to meet the mean diameter criterion of the hair cosmetic emulsion of the present invention.

The hair cosmetic emulsions of the invention can also be prepared using a microfluidic method, in particular such as described in international applications WO2012/120043 or WO2015/055748.

Preferably, the viscosity of the oil phase (OP) of the droplets of hair cosmetic emulsion is between 20 000 and 10 000 000 mPas, preferably between 50 000 and 1 000 000 mPas, and better still between 100 000 and 500 000 mPas, at 25° C. The viscosity is measured using the above-described method.

In a preferred embodiment the oil phase (OP) of the hair cosmetic emulsion comprises at least one gelling agent.

The term "at least one gelling agent" means exactly one gelling agent and, also a mixture of two or more different gelling agents.

As indicated above, the oil phase (OP) according to the present invention comprises preferably at least one gelling agent. Said gelling agent differs from the anionic polymers described above. In the invention and unless otherwise stated, by "gelling agent" it is meant an agent allowing an increase in the viscosity of the oil phase (OP) of the droplets of the hair cosmetic emulsion by said gelling agent, and allowing the oil phase (OP) to reach a viscosity higher than 20 000 mPas, preferably higher than 50 000 mPas, more preferably higher than 100 000 mPas, and further preferably higher than 200 000 mPas.

Preferably, the viscosity of the oil phase (OP) of the droplets of the hair cosmetic emulsion in the presence of said gelling agent is between 20 000 and 100 000 000 mPas, preferably between 50 000 and 1 000 000 mPas, and better still between 100 000 and 500 000 mPas, at 25° C.

In a preferred embodiment, the gelling agent is an ester of glycerol and fatty acid(s), in particular a mono-, di- or triester of glycerol and fatty acid(s). Typically, said ester of glycerol and fatty acid(s) can be used alone or in a mixture. As gelling agent according to the invention an ester of glycerol and of a fatty acid, or an ester of glycerol and a mixture of fatty acids can be used.

In a preferred embodiment, the fatty acid is selected from the group formed by behenic acid, isooctadecanoic acid, stearic acid, eicosanoic acid, and mixtures thereof.

In an especially preferred embodiment, the gelling agent is an ester of glycerol and fatty acid(s) of the formula: 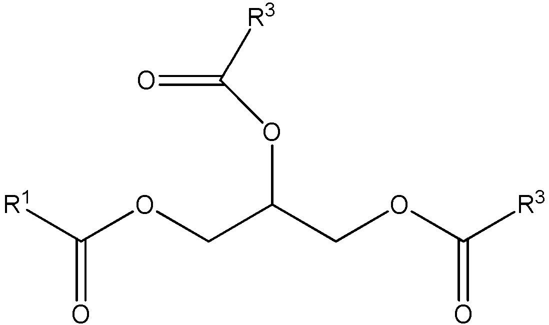 wherein
R¹, R² and R³ are each independently selected from among H and a saturated alkyl chain having 4 to 30 carbon atoms, wherein at least one of R¹, R² and R³ differing from H.

In one embodiment, R¹, R² and R³ are different.

In one embodiment, R¹, R² and/or R³ are a saturated alkyl chain having from 4 to 30 carbon atoms, preferably 12 to 22 and more preferably 18 to 22 carbon atoms.

In one embodiment, the ester of glycerol and fatty acid(s) corresponds to a compound of the above formula, wherein R¹ is H, R² is C₂₁H₄₃ and R³ is C₁₉H₄₀.

In one embodiment, the ester of glycerol and fatty acid(s) corresponds to a compound of the above formula, wherein R¹, R² and R³ are C₂₁H₄₃.

In one embodiment, the ester of glycerol and fatty acid(s) corresponds to a compound of the above formula, wherein R¹ and, R² are H, and R³ is C₁₉H₄₀.

In one embodiment, the ester of glycerol and fatty acid(s) corresponds to a compound of the above formula, wherein R¹ and R² are H, and R³ is C₁₇H₃₅.

Particular mention can be made of the esters of glycerol and fatty acid(s) marketed under the trade names Nomcort HK-G (INCI name: Glyceryl behenate/eicosadioate) and Nomcort SG (INCl name: Glyceryl tribehenate, isostearate, eicosadioate), by Nisshin Oillio and THIXCIN^{®} R by Elementis Specialties (INCI: Trihydroxystearin) or Estogel E by PolymerExpert (Proposed INCI name: caprylic/capric triglyceride, castor oil/IPDI copolymer). caprylic/capric triglyceride, castor oil/IPDI copolymer is also available under the trade name EMC30 from PolymerExpert.

Another suitable gelling agent is coco-caprylate/caprate form BASF sold under the tradename Cetiol C5C.

In an especially preferred embodiment, the gelling agent is a mixture of coco-caprylate/caprate and caprylic/capric triglyceride, castor oil/IPDI copolymer.

The oil phase (OP) of the hair cosmetic emulsion comprises preferably 10 to 50 wt.%, more preferably 20 to 40 wt.%, and most preferred 29 to 37 wt.%, of oil soluble botanicals, based on the total weight of the oil phase (OP) comprised in the hair cosmetic emulsion.

The oil soluble botanicals are preferably components b3), b4), b5), b6), b7), b8), and b9) as described above.

The oil phase (OP) of the hair cosmetic emulsion, moreover, comprises preferably 90 to 50 wt.%, more preferably 80 to 60 wt.%, and most preferred 71 to 63 wt.%, of stabilizing agents.

The stabilizing agents are preferably the additional components described herein, which might be present in the oil phase (OP).

In one embodiment the hair cosmetic emulsion may comprise one or more of the following components.

For example, one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol (provitamin B5), omega-3 fatty acids, omega-6 fatty acids, folates, cannabidiol, amino acids, or combinations thereof.

The hair cosmetic emulsion according to the present invention optionally may comprise cosmetically acceptable additives According to embodiments, the cosmetically acceptable additives may be selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents. The cosmetically acceptable additives may be used in any combinations, as desired or required.

Suitable cosmetically acceptable additives not specifically described below are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific additives to achieve a particular purpose or multipurpose. A few of these additives are discussed below, whose invention is of course non-exhaustive.

The hair cosmetic product according to the present invention may comprise a preservative or mixture of preservatives. The hair cosmetic product may comprise the preservative(s) in an amount of up to 1.0, for example from 0.0010 to 1.0, in particular from 0.010 to 0.50 percent-by-weight, based on the weight of the hair cosmetic product. Cosmetically acceptable preservatives include organic acids such as para-hydroxybenzoic acid; organic acid salts such as sodium benzoate; compounds such as benzyl alcohol, phenoxyethanol, 1,3-bis(hydroxymethyl)-5,5-dimethylimidazolidine-2,4-dione; and mixtures thereof.

The according to the present invention may further comprise one or more chelators (also known as "chelating agent", "sequestering agent", "sequestrant", or chelants) in an amount sufficient to reduce the amount of metals available to interact with formulation components. Chelators are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996). Typically, the compositions may comprise one or more chelators in a total amount of from 0.0010 to 1.0, in particular from 0.010 to 0.50 percent-by-weight, based on the weight of the.

For example the hair cosmetic emulsion may comprise one or more chelators selected from aminocarboxylic acid chelators comprising one or more carboxylic acid moieties (-COOH) and one or more nitrogen atoms. The one or more aminocarboxylic acid chelators may be selected from the group consisting of diethylenetriamine pentaacetic acid (DTPA), ethylenediamine disuccinic acid (EDDS), ethylenediamine-N,N'-diglutaric acid (EDDG), 2-hydroxypropylenediamine-N-N'-disuccinic acid (HPDDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), ethylenedicysteic acid (EDC), ethylenediamine-N-N'-bis(ortho-hydroxyphenyl acetic acid) (EDDHA), diaminoalkyldi(sulfosuccinic acids) (DDS), N,N'-bis(2-hydroxybenzyl)ethylenediamine-N,N'-diacetic acid (HBED), their salts thereof, and mixtures thereof. Conveniently, a chelator used in compositions according to the present invention, if any, may be EDTA.

In one embodiment the hair cosmetic product according to the present invention may be essentially free of, or be free of certain substances or classes of chemical compounds.

In an especially preferred embodiments, the hair cosmetic product according to the present invention may be essentially free of silicones. More preferred the hair cosmetic product according to the present invention may be free of silicones. According to particular embodiments, the concentration of silicones may be less than 5 ppm by weight based on the weight of the hair cosmetic product.

Another aspect of the present invention is a method for treating hair comprising applying the hair cosmetic emulsion according to the invention to a person's scalp hair.

Another aspect of the present invention it the use of the hair cosmetic emulsion product according to the invention for treating human hair.

Yet another aspect of the present invention is the use of the hair cosmetic emulsion according to the invention for nourishing and/or strengthening human hair.

Yet another aspect of the present invention is the use of the hair cosmetic emulsion according to the invention for to protect human hair from pollution.

### Embodiments

In the following, there is provided a list of embodiments to further illustrate the present invention without intending to limit the invention to the specific embodiments listed below.
1. Hair cosmetic emulsion comprising
   an aqueous phase (AP) forming the continuous phase of the hair cosmetic emulsion, and
   an oil phase (OP) forming the disperse phase of the hair cosmetic emulsion,
   wherein the hair cosmetic emulsion comprises a botanical mixture (BM) comprising the following 9 botanical components
      - b1): bamboo extract,
      - b2): cactus extract,
      - b3): jojoba oil,
      - b4): safflower oil,
      - b5): olive oil,
      - b6): babassu oil,
      - b7): sesame seed oil,
      - b8): rice bran oil, and
      - b9): evening primrose oil.
2. Hair cosmetic emulsion according to embodiment 1, wherein the botanical mixture (BM) comprises
   1 to 10 % by weight of component b1),
   1 to 10 % by weight of component b2),
   10 to 30 % by weight of component b3),
   10 to 30% by weight of component b4),
   5 to 15 % by weight of component b5),
   5 to 15 % by weight of component b6),
   5 to 15 % by weight of component b7),
   5 to 15 % by weight of component b8),
   5 to 15 % by weight of component b9),
   wherein the % by weight values are based on the total weight of the botanical mixture (BM) comprised in the hair cosmetic emulsion, wherein the sum of components b1) to b9) is less than or equal to 100 % by weight of the botanical mixture (BM).
3. A hair cosmetic emulsion according to embodiment 1 or 2, wherein the hair cosmetic emulsion comprises 0.5 wt.-% to 5.0 % by weight of the botanical mixture (BM) based on the total weight of the hair cosmetic emulsion.
4. A hair cosmetic emulsion according to any one of embodiments 1 to 3, wherein the botanical components b1) and b2) are comprised in the aqueous phase (AP) of the hair cosmetic emulsion and the botanical components b3), b4), b5), b6), b7), b8), and b9) are comprised in the oil phase (OP) of the hair cosmetic emulsion.
5. A hair cosmetic emulsion according to any one of embodiments 1 to 4, wherein the oil phase (OP) comprised in the hair cosmetic emulsion is in the form of droplets.
6. A hair cosmetic emulsion according to embodiment 5, wherein the droplets have an average diameter of in the range of 100 to 3000 µm.
7. A hair cosmetic emulsion according to any one of embodiments 1 to 6, wherein the pH value of the aqueous phase (AP) and/or the oil phase (OP) is in the range of 5.0 to 7.0, preferably in the range of 5.5 to 6.5.
8. A hair cosmetic emulsion according to any one of embodiments 1 to 7, wherein the hair cosmetic emulsion comprises
   95.0 to 99.5 wt.-% of the aqueous phase (AP), and
   0.5 to 5.0 wt.-% of the oil phase (OP),
   based on the total weight of the hair cosmetic emulsion.
9. A hair cosmetic emulsion according to any one of embodiments 1 to 8, wherein the aqueous phase (AP) of the hair cosmetic emulsion comprises at least one anionic polymer in an amount in the range of 0.05 to 1.0 wt.-% based on the total weight of the aqueous phase (AP) comprised in the hair cosmetic emulsion.
10. A hair cosmetic emulsion according to any one of embodiments 1 to 9, wherein the aqueous phase (AP) of the hair cosmetic emulsion comprises at least one polyvinylpyrrolidone thickener in an amount in the range of 0.1 to 1.5 wt.-% based on the total weight of the aqueous phase (AP) comprised in the hair cosmetic emulsion.
11. A hair cosmetic emulsion according to any one of embodiments 1 to 10, wherein the oil phase (OP) of the hair cosmetic emulsion comprises at least one gelling agent in an amount in the range of 30 to 80.0 wt.-% based on the total weight of the oil phase (OP) comprised in the hair cosmetic emulsion.
12. A hair cosmetic emulsion according to any one of embodiments 1 to 11, wherein the aqueous phase (AP) of the hair cosmetic emulsion comprises water in an amount of 70 to 90 wt.-% based on the total weight of the aqueous phase (AP) comprised in the hair cosmetic emulsion.
13. A hair cosmetic emulsion according to any one of embodiments 1 to 12, wherein the aqueous phase (AP) of the hair cosmetic emulsion comprises at least one alcohol in an amount of 10 to 20 wt.-% based on the total weight of the aqueous phase (AP) comprised in the hair cosmetic emulsion.
14. The hair cosmetic emulsion according to any one of embodiments 1 to 13, wherein the hair oil comprises at least one of the following ingredients of antioxidants, vitamins, UV-filters, fragrances and/or preservatives.
15. The hair cosmetic emulsion according to any one of embodiments 1 to 14, wherein the hair oil comprises tocopherol, and/or panthenol.
16. The hair cosmetic emulsion according to any one of embodiments 1 to 15, wherein the at least one conditioning agent is at least one selected from the group consisting of dimethicone, cyclopentasiloxane, behentrimonium chloride, cetrimonium chloride, polyquaternium 6, and polyquaternium 10.
17. The hair cosmetic emulsion according to any one of embodiments 1 to 16, wherein the emollient is at least one emollient selected from the group consisting of argan oil, coconut oil, almond oil, squalane, squalene, ethyl hexyl palmitate, cetyl ethylhexaloate, and octyldodecanol.
18. The hair cosmetic emulsion according to any one of embodiments 1 to 17, wherein the hair cosmetic product comprises one more or all of the following components
   a) at least one C3-C13 saturated or unsaturated dicarboxylic acid, which is unsubstituted or substituted with one hydroxyl group,
   b) at least one C10-C30 saturated or unsaturated monocarboxylic,
   c) at least one C10-C40 saturated or unsaturated hydrocarbon,
   d) at least one fatty acid ester of a C10-C30 saturated or unsaturated monocarboxylic acid and a C3-C6 mono-ol, diol or triol.
19. The hair cosmetic emulsion according to embodiment 18, wherein component a) is at least one C3-C6 saturated or unsaturated dicarboxylic acid, the saturated dicarboxylic acid being optionally substituted with one hydroxyl group.
20. The hair cosmetic emulsion according to embodiment 18 or 19, wherein the component a) is selected from the group consisting of malonic acid, succinic acid, glutaric acid, adipic acid, malic acid, hydroxysuccinic acid, hydroxyglutaric acid, fumaric acid, maleic acid, itaconic acid, glutaconic acid, salts thereof, and any combination thereof.
21. The hair cosmetic emulsion according to any one of embodiments 18 to 20, wherein component b) is at least one C16-C22 saturated or unsaturated monocarboxylic acid.
22. The hair cosmetic emulsion according to any one of embodiments 18 to 21, wherein component d) is at least one fatty acid ester of a C16-C22 saturated or unsaturated monocarboxylic acid and a C3-C6 triol.
23. The hair cosmetic emulsion according to any one of embodiments 18 to 22, wherein component c) is at least one C26-C40 saturated or unsaturated hydrocarbon.
24. The hair cosmetic emulsion according to any one of embodiments 18 to 23, wherein
   component a) is at least one selected from the group consisting of malic acid and hydroxyglutaric acid,
   component b) is at least one selected from the group consisting of oleic acid and palmitoleic acid,
   component c) is at least one selected from the group consisting of squalane and squalene,
   component d) is at least one selected from the group consisting of glyceryl monooleate and glyceryl mono-palmitoleate.
25. The hair cosmetic emulsion according to any one of embodiments 1 to 24, wherein the hair cosmetic product comprises less than 5ppm by weight of a silicon containing component
26. The hair cosmetic emulsion according to any one of embodiments 1 to 25, wherein the hair cosmetic product comprises does not contain any silicon containing component(s).
27. The hair cosmetic emulsion according to any one of embodiments 1 to 26, wherein the oil phase (OP) of the hair cosmetic emulsion comprises preferably 10 to 50 wt.%, more preferably 20 to 40 wt.%, and most preferred 29 to 37 wt.%, of oil soluble botanicals, based on the total weight of the oil phase (OP) comprised in the hair cosmetic emulsion.
28. The hair cosmetic emulsion according to embodiment to 27, wherein the oil soluble botanicals are preferably components b3), b4), b5), b6), b7), b8), and b9).
29. The hair cosmetic emulsion according to embodiment 27 or 28, wherein the oil phase (OP) of the hair cosmetic emulsion, moreover, comprises preferably 90 to 50 wt.%, more preferably 80 to 60 wt.%, and most preferred 71 to 63 wt.%, of at least one stabilizing agents.
30. The hair cosmetic emulsion according to embodiment to 29, wherein the at least one stabilizing agent is selected from the additional components optionally present in the oil phase (OP).
31. A method for treating hair comprising applying the hair cosmetic emulsion according to any one of claims 1 to 30 to a person's scalp hair.
32. Use of the hair cosmetic emulsion according to any one of claims 1 to 31 to protect the hair from pollution.

### EXAMPLES

The following examples illustrate the formulations and performance results of the hair cosmetic emulsion comprising the botanical mixture (BM) according to the presently claimed invention. These examples are not intended to be inclusive of all aspects of the subject matter disclosed herein, but rather to illustrate representative compositions and results. These examples are not intended to exclude equivalents and variations of the presently claimed invention, which are apparent to one skilled in the art.

The following hair cosmetic emulsion was tested.

HT1 is hair cosmetic emulsion according to the invention. The components contained in the hair cosmetic emulsion HT1 are given in table 1 below.

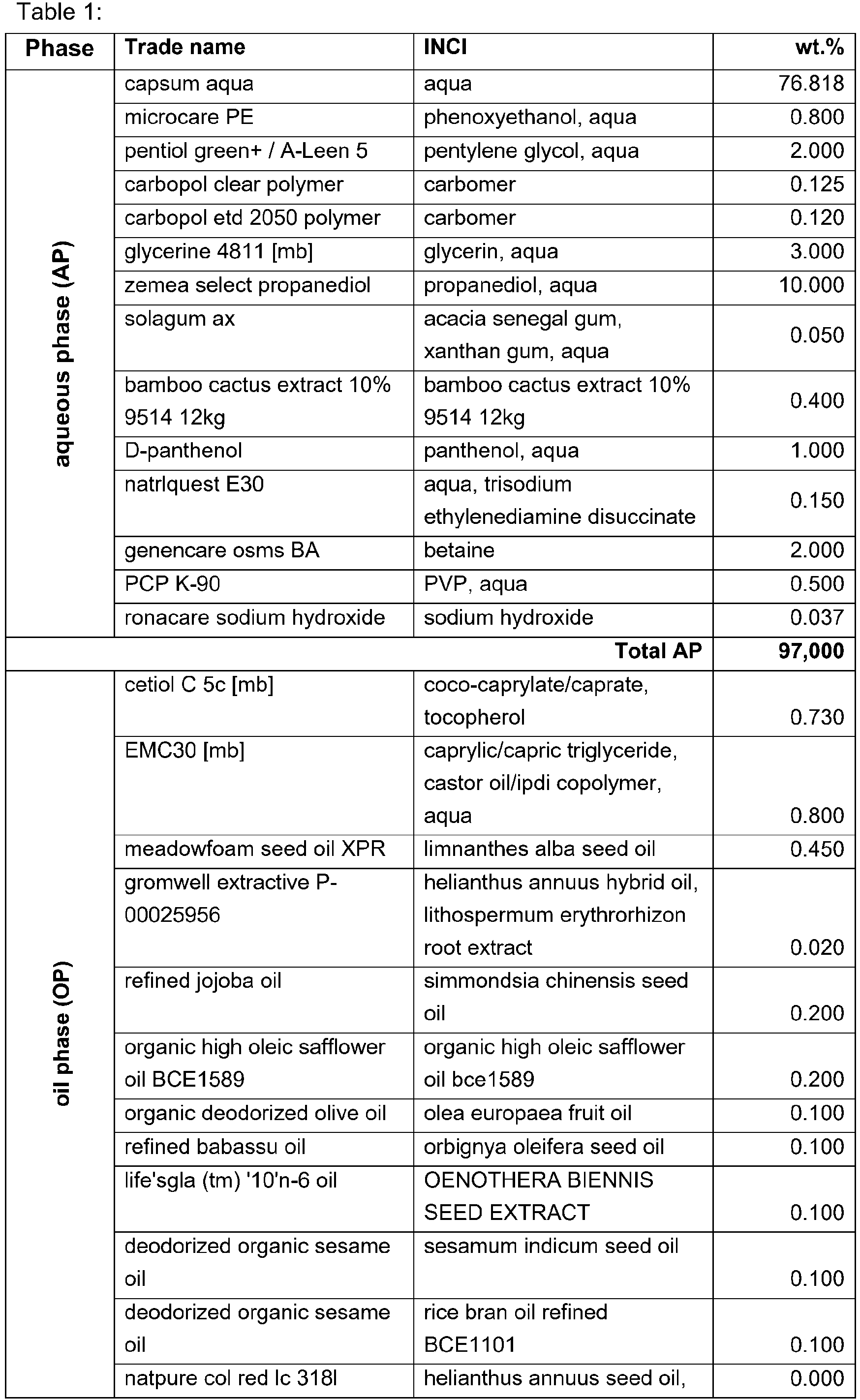

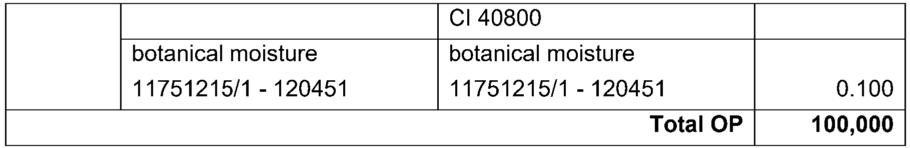

All testing is performed using natural white, blond hair tresses supplied by International Hair Importers & Products (Glendale, NY). The hair tresses weigh approximately 3 g and measure 8" in length and 1" in width. Ten (10) hair tresses are used per treatment group.

All treatment is performed using an intellifaucet set at 40°C with controlled flow rate of 1 GPM. Before the experiment, all tresses are standardized using a 15% sodium laureth sulfate SLES solution.

### Treatment group (TG0):

TG0 is the water control group, no hair cosmetic emulsion is used. One cycle, (1 cycle) is carried out.

### Treatment group (TG1):

In TG1 the hair cosmetic emulsion HT1 (according to the invention) is used. On dry hair, the hair cosmetic emulsion is applied at 10% by weight of the tress (0.1g/g of hair), massage 30 seconds (ensure droplets are dissolved), the hair cosmetic emulsion is left in and not rinsed. One cycle (1 cycle) is carried out.

Applications:
1. Baseline measurements are taken on clean, untreated hair.
2. Tresses are treated with test product as outlined above and allowed to dry and equilibrate.
3. Tresses are exposed to pollution as outlined below.
4. Post-pollution measurements are taken.
5. Tresses are treated again with 15% SLES solution and allowed to dry and equilibrate.
6. Post-wash measurements are taken.

### Exposure to Particle Pollution

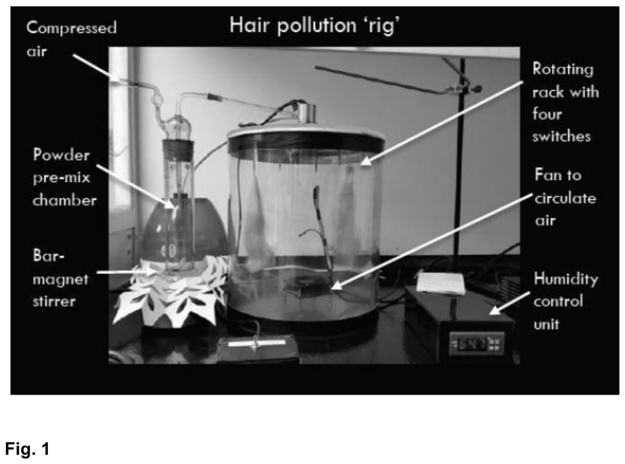

A test chamber, see enclosed figure 1, is the basis of the protocol. Four tresses are exposed at a time.
1. Turn on the power using the power control unit. Take out the glass tubing that connects the premix and main chambers. Open the lid of the main chamber. Brush out four hair tresses and hang them on the rack inside the main chamber (every second clip). Close the lid. Place the glass tubing so it will connect the pre-mix and main chambers. Open the compressed air valve, and using the compressed air flow, let air run for 5 minutes to dry the chamber. Reduce gas flow to the minimum and turn off the compressed air valve.
2. Weigh 25 mg of graphite particles (approximately 1 µm particle size) and place in the pre-mix chamber. Use a magnetic stirrer to break up large particle agglomerates.
3. Use the hair tress rotation control panel to start the rotation of tresses. The knob allows the control of the rotation direction (clockwise/counterclockwise). The rotational direction will be reversed every two minutes throughout the exposure period for more even particle distribution on the hair surface.
4. Open the compressed air valve and let gas flow slowly so particles will be introduced in the main chamber gradually (during the first 10 min no particles should remain in the pre-mix chamber). Compressed air should keep the relative humidity of the main chamber below 10%. After 18 minutes, reduce compressed air flow to the minimum and turn off the valve and magnetic stirrer. Turn on humidifier at maximum capacity so the humidity of the chamber increases to approximately 50%. After 2 minutes (20 minutes total), stop the rotation of tresses and leave tresses in the chamber for 5 minutes to reduce contamination of environment after the lid is open.
5. Take tresses out and brush them 10 times to remove tangles prior to measurements (use different brushes for different treatments).

The evaluations are performed after one cycle.

All the hair tresses are labeled. Baseline L, a, b measurements are taken using a WF30 hand-held colorimeter to characterize the color of hair.

Technical hair color is quantified using the CIELAB L, a, b system. That color is represented in a 3-dimensional matrix where "L" refers to the lightness on a scale of 0 to 100, "a" denotes the red-green color range (positive value denotes higher red) and "b" represents the yellow-blue color range (positive value denotes higher yellow).

After one cycle of pollution, there is statistical differenced in lightness, delta L, between treatment group TG0 (water control) and treatment group TG1. The lightness values, delta L, are given in table 2 below.

**Table 2 (delta L after pollution):**

| Treatment | N | Mean | Std Dev | Std Err Mean | Level | |
|---|---|---|---|---|---|---|
| TG0 (water control) | 10 | -30.9 | 1.6 | 0.5 | A | |
| TG1 (Treatment HT1) | 10 | -40.7 | 1.8 | 0.6 | | C |

After washing (post wash), there is statistical differenced in lightness, delta L, between treatment group TG0 (water control) and treatment group TG1. The lightness values, delta L, are given in table 3 below.

**Table 3 (delta L post wash):**

| Treatment | N | Mean | Std Dev | Std Err Mean | Level | |
|---|---|---|---|---|---|---|
| TG1 (Treatment HT1) | 10 | -13.5 | 1.6 | 0.5 | A | |
| TG0 (water control) | 10 | -21.6 | 2.7 | 0.9 | | B |

The post wash results shown in table 3 show a statistically lower change in lightness showing that the inventive hair cosmetic emulsion makes it easier to remove particulate pollution from the surface of the hair with washing.

## Claims

1. Hair cosmetic emulsion comprising
an aqueous phase (AP) forming the continuous phase of the hair cosmetic emulsion, and
an oil phase (OP) forming the disperse phase of the hair cosmetic emulsion,
wherein the hair cosmetic emulsion comprises a botanical mixture (BM) comprising the following 9 botanical components
b1) bamboo extract,
b2) cactus extract,
b3) jojoba oil,
b4) safflower oil,
b5) olive oil,
b6) babassu oil,
b7) sesame seed oil,
b8) rice bran oil, and
b9) evening primrose oil.

2. Hair cosmetic emulsion according to claim 1, wherein the botanical mixture (BM) comprises
1 to 10% by weight of component b1),
1 to 10 % by weight of component b2),
10 to 30 % by weight of component b3),
10 to 30% by weight of component b4),
5 to 15 % by weight of component b5),
5 to 15 % by weight of component b6),
5 to 15 % by weight of component b7),
5 to 15 % by weight of component b8),
5 to 15 % by weight of component b9),
wherein the % by weight values are based on the total weight of the botanical mixture (BM) comprised in the hair cosmetic emulsion, wherein the sum of components b1) to b9) is less than or equal to 100 % by weight of the botanical mixture (BM).

3. A hair cosmetic emulsion according to claim 1 or 2, wherein the hair cosmetic emulsion comprises 0.5 wt.-% to 5.0 % by weight of the botanical mixture (BM) based on the total weight of the hair cosmetic emulsion.

4. A hair cosmetic emulsion according to any one of claims 1 to 3, wherein the botanical components b1) and b2) are comprised in the aqueous phase (AP) of the hair cosmetic emulsion and the botanical components b3), b4), b5), b6), b7), b8), and b9) are comprised in the oil phase (OP) of the hair cosmetic emulsion.

5. A hair cosmetic emulsion according to any one of claims 1 to 4, wherein the oil phase (OP) comprised in the hair cosmetic emulsion is in the form of droplets.

6. A hair cosmetic emulsion according to claim 5, wherein the droplets have an average diameter of in the range of 100 to 3000 µm.

7. A hair cosmetic emulsion according to any one of claims 1 to 6, wherein the pH value of the aqueous phase (AP) and/or the oil phase (OP) is in the range of 5.0 to 7.0, preferably in the range of 5.5 to 6.5.

8. A hair cosmetic emulsion according to any one of claims 1 to 7, wherein the hair cosmetic emulsion comprises
95.0 to 99.5 wt.-% of the aqueous phase (AP), and
0.5 to 5.0 wt.-% of the oil phase (OP
based on the total weight of the hair cosmetic emulsion.

9. A hair cosmetic emulsion according to any one of claims 1 to 8, wherein the aqueous phase (AP) of the hair cosmetic emulsion comprises at least one anionic polymer comprising carboxylic acid functions -COOH, which might be present in the form of an carboxylate anion -COO⁻, in an amount in the range of 0.05 to 1.0 wt.-% based on the total weight of the aqueous phase (AP) comprised in the hair cosmetic emulsion.

10. A hair cosmetic emulsion according to any one of claims 1 to 9, wherein the aqueous phase (AP) of the hair cosmetic emulsion comprises at least one polyvinylpyrrolidone thickener in an amount in the range of 0.1 to 1.5 wt.-% based on the total weight of the aqueous phase (AP) comprised in the hair cosmetic emulsion.

11. A hair cosmetic emulsion according to any one of claims 1 to 10, wherein the oil phase (OP) of the hair cosmetic emulsion comprises at least one gelling agent in an amount in the range of 30 to 80.0 wt.-% based on the total weight of the oil phase (OP) comprised in the hair cosmetic emulsion.

12. A hair cosmetic emulsion according to any one of claims 1 to 11, wherein the aqueous phase (AP) of the hair cosmetic emulsion comprises water in an amount of 70 to 98 wt.-% based on the total weight of the aqueous phase (AP) comprised in the hair cosmetic emulsion.

13. A hair cosmetic emulsion according to any one of claims 1 to 12, wherein the aqueous phase (AP) of the hair cosmetic emulsion comprises at least one alcohol in an amount of 10 to 20 wt.-% based on the total weight of the aqueous phase (AP) comprised in the hair cosmetic emulsion.

14. A method for treating hair comprising applying the hair cosmetic emulsion according to any one of claims 1 to 13 to a person's scalp hair.

15. Use of the hair cosmetic emulsion according to any one of claims 1 to 13 to protect the hair from pollution.
